# EUROPÄISCHE PATENTSCHRIFT

(11) **EP 1 308 135 B1**
(45) Veröffentlichungstag und Bekanntmachung des Hinweises auf die Patenterteilung: **24.11.2004**
(21) Anmeldenummer: 02021293.2
(22) Anmeldetag: 19.09.2002
(51) Int. Cl.: A61B 17/80

(54) **Vorrichtung zur Stabilisierung der Tibia und/oder Tibiakopfes nach Osteotomie-Eingriffen**
Device for stabilising the tibia and/or the head of the tibia after osteotomy surgery
Dispositif pour stabiliser le tibia et/ou la tête de tibia suite à une ostéotomie

(30) Priorität: 30.10.2001 DE 10153467
(43) Veröffentlichungstag der Anmeldung: 07.05.2003
(73) Patentinhaber: Königsee Implantate und Instrumente zur Ostheosynthese GmbH, 07426 Königsee (DE)
(72) Erfinder: Spahn, Gunter, Dr.med., 99831 Creuzburg (DE); Prochazka, Sylvia, 07407 Rudolstadt (DE)
(74) Vertreter: Kruspig, Volkmar, Dipl.-Ing.

(56) Entgegenhaltungen:
- WO-A-96/14802
- DE-A- 10 015 734
- FR-A- 2 727 005
- FR-A- 2 785 519

## Beschreibung

Die Erfindung betrifft eine Vorrichtung zu Stabilisierung der Tibia und/oder des Tibiakopfes nach Osteotomie-Eingriffen, bestehend aus einem langgestreckten Plattenkörper mit Bohrungen zur Aufnahme von Knochenschrauben sowie einem quaderförmigen Platz- oder Abstandshalter, gemäß Oberbegriff des Patentanspruchs 1.

Unter Osteotomie wird eine lineare quer-, schräg-, bogen-, zylinder- oder V- bzw. keilförmige Knochendurchtrennung, meist mit anschließender Osteosynthese entweder als vollständige Osteotomie nach Freilegen und Abschieben des Periosts oder als weiche Osteotomie mit Teildurchmeißelung und nachfolgender Frakturierung verstanden. Die Osteotomie findet Anwendung zur Verkürzung, Verlängerung, Achsenumstellung, Rotation oder Abstützung, z.B. als valgisierende und varisierende Osteotomie am koxalen Femurende.

Aus den US-Patentschriften 5,620,448 und 5,749,875 ist ein Knochenplattensystem für die sogenannte Open Wedge Osteotomie vorbekannt.

Die dortige Platte weist eine langgestrecke Form mit an den jeweiligen Enden gegenüberliegenden Befestigungsbohrungen für Knochenschrauben auf.
Die Platte selbst besitzt eine Erhöhung mit unterschiedlichen Breitenmaßen.

Nach Ausführen des Osteotomie-Schnitts und Setzen eines gabelartigen Spreizwerkzeugs wird die aus den zitierten US-Patenten bekannte Platte mit ihrem Fortsatz als Platz- oder Abstandshalter gesetzt und anschließend mit Knochenschrauben an der Tibia fixiert.

Es hat sich jedoch gezeigt, daß durch die hohen punktuellen Belastungen beim Aufspreizen und Keilsetzen Spontanfrakturen, insbesondere des Tibiakopfes auftreten, verbunden mit dem hierdurch einhergehenden Operationsrisiko. Insofern ist es vielfach erforderlich, vorsorglich oder im Nachgang Fixationsplatten oder -schrauben am Tibiakopf zu setzen.

Dadurch, daß der Plattenkörper nach US-PS 5,620,448 einen geometrisch festen, unveränderbaren Bezug zwischen Auskragung als Platz- oder Abstandshalter und den Bohrungen zur Aufnahme der Knochenschrauben besitzt, ist es nicht möglich, eine gegebenenfalls günstigere Position zum Setzen der Knochenschrauben auszuwählen.

Da der bekannte Plattenkörper nebst Platz- und Abstandshalter einstückig ausgebildet ist, muß stets ein Satz von verschiedenen Platten mit unterschiedlichen Abmessungen im Bereich des Platzhalters vorrätig gehalten werden, was zu höheren Kosten führt.

Dokument FR 2 785 519 A offenbart eine Vorrichtung nach dem Oberbegriff des Anspruchs 1.

Aus dem Vorgenannten ist es daher Aufgabe der Erfindung, eine weiterentwickelte Vorrichtung zur Stabilisierung der Tibia und/oder des Tibiakopfes nach Osteotomie-Eingriffen anzugeben, die bzw. das es gestattet, im vorgebbaren Rahmen günstige Befestigungspositionen für die eigentliche Knochenplatte zu finden, und wobei in einfacher Weise eine einzige Knochenplatte mit verschiedenen Abstandshaltern, d.h. Abstandshaltern unterschiedlicher Maße Verwendung finden kann.

Die Lösung der Aufgabe der Erfindung erfolgt vorrichtungsseitig mit einem Gegenstand nach den Merkmalen des Patentanspruchs 1, wobei die Unteransprüche mindestens zweckmäßige Ausgestaltungen und Weiterbildungen darstellen.

Erfindungsgemäß weist die Knochenplatte oder der Plattenkörper ein in Längsachsenrichtung verlaufendes Langloch auf, wobei ein jeweiliger Platz- oder Abstandshalter verschieblich und feststellbar über eine Schraubverbindung im Langloch des Plattenkörpers gehalten ist.

Der Plattenkörper besitzt bevorzugt eine T-Form und die Platz- oder Abstandshalter sind austauschbar und weisen unterschiedliche Höhenabmessungen je nach Korrekturwinkel auf.

Der Platz- oder Abstandshalter besitzt eine U-förmige Ausnehmung, wobei die seitlichen, relativ kurzen Schenkel der Ausnehmung den Plattenkörper jeweils seitlich umgreifen, wodurch eine Verdrehsicherung und Führung beim Verschieben im Langloch des Plattenkörpers gebildet ist.

Zum Befestigen besitzt der Platz- oder Abstandshalter eine Gewindebohrung, die sich im Verbindungsabschnitt zwischen den seitlichen Schenkeln der Ausnehmung befindet.

Das Langloch kann zur Oberseite des Plattenkörpers hin gerichtet eine konische Fase, die umlaufend ausgebildet ist, aufweisen.

Die Schraube zur Befestigung des Platz- oder Abstandshalters weist einen sich konisch verjüngenden Schraubenkopf oder Schraubenkopfabschnitt auf.

Im querliegenden Kopfteil des T-förmigen Plattenkörpers sind mindestens zwei Befestigungsbohrungen sowie im langgestreckten Fußteil dem Langloch in Längsachsenrichtung benachbart jeweils mindestens eine Befestigungsbohrung ausgebildet. Bei unterschiedlichen Längenmaßen des Fußteils kann die Anzahl der Befestigungsbohrungen erhöht oder aber auch verringert sein.

Die Kopfteilbohrungen dienen einerseits der Befestigung des Plattenkörpers im Tibiakopf und andererseits aber auch zum Stabilisieren der Knochenstruktur des Kopfes selbst. Um den Heilungsprozeß zu fördern, besitzt der Querschnitt des Plattenkörpers eine Bogen- oder Kreissegmentform, wobei der Plattenkörper so gesetzt ist, daß er bezogen auf die Knochenoberfläche konvex angeordnet wird.

Die beschriebene Vorrichtung zur Stabilisierung der Tibia und/oder des Tibiakopfes nach Osteotomie-Eingriff wird wie folgt verwendet.
Zunächst wird nach Schnittführung und Setzen mindestens eines Spreizkeils je nach Korrekturmaß ein höhenmäßig entsprechender Platz- oder Abstandshalters ausgewählt und dieser am Plattenkörper verschieblich befestigt, und zwar unter Zuhilfenahme der entsprechenden Befestigungsschraube.

Im Anschluß darin wird der Plattenkörper mit anmontiertem Platz- oder Abstandshalter an der Tibia plaziert und hierbei der Platzhalter in den offenen Ostoetomie-Keilschnitt eingesetzt. Aufgrund der gegebenen Beweglichkeit des Plattenkörpers kann nun eine optimale Befestigungsposition und -lage für die Befestigungsbohrungen, die im Plattenkörper vorgesehen sind, bestimmt werden. Nachdem die aus der Sicht des Operateurs vorteilhafte Position ermittelt wurde, wird mit bevorzugt selbstschneidenden Schrauben der Plattenkörper an der Tibia und dem Tibiakopf fixiert. Ebenso erfolgt ein Anziehen der Schraube zum endgültigen Befestigen des Platzhalters.

Die Konstruktion des T-förmigen Plattenkörpers mit Befestigungsbohrungen, die möglichst weit weg vom aufgetrennten Knochenteil befindlich sind, reduziert die Gefahr unerwünschter weiterer Belastungen im Knochen und fördert den Heilungsprozeß.

Die Erfindung soll nachstehend anhand eines Ausführungsbeispiels sowie unter Zuhilfenahme von Figuren näher erläutert werden.

Hierbei zeigen:
- Fig. 1: verschiedene Ansichten des langgestreckten Plattenkörpers mit und ohne befestigten Platz- oder Abstandshalter und

Fig. 2 eine Darstellung von Femur und Tibia mit ausgeführter keilförmiger Osteotomie und dem zu setzenden Plattenkörper mit Platz- oder Abstandshalter.

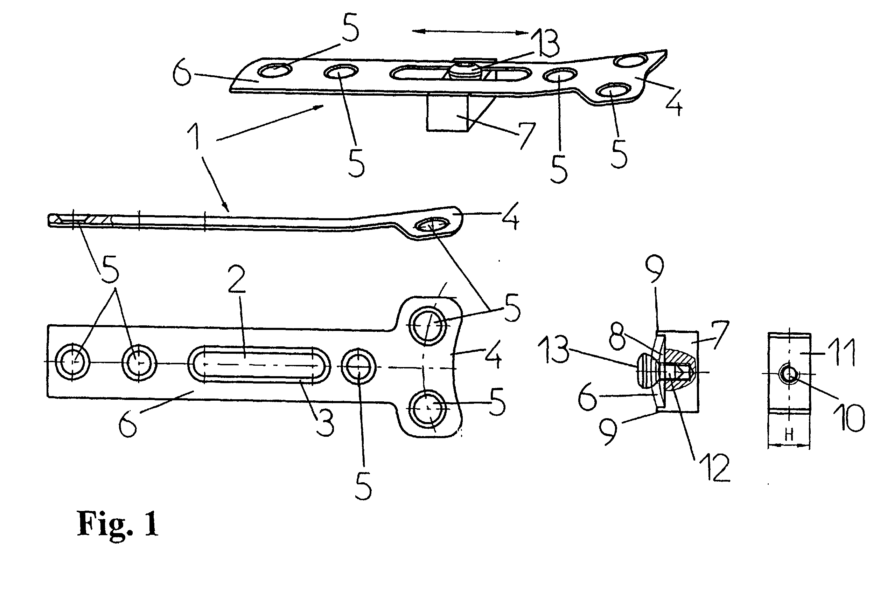

Der erfindungsgemäße Plattenkörper 1 weist, wie in der Fig. 1 ersichtlich, eine langgestreckte T-Form mit einem leicht gewölbten bzw. kreisbogenförmigen Querschnitt auf.

Die Draufsicht im linken unteren Bildteil läßt das in Längsrichtung verlaufende Langloch 2 und die dort ausgebildete umlaufende konische Fase 3 erkennen.

Kopfseitig, d.h. im querliegenden Kopfteil 4 des Plattenkörpers 1 sind zwei beabstandete Befestigungsbohrungen 5 vorgesehen. Weitere Befestigungsbohrungen befinden sich dem Langloch 2 benachbart im Fußteil 6 des Plattenkörpers 1.

Der austauschbar gestaltete quaderförmige Platz- oder Abstandshalter 7 besitzt eine U-förmige Ausnehmung 8 dergestalt, daß die seitlichen Schenkel 9 der U-förmigen Ausnehmung eine Verdrehsicherung und Führung in Verbindung mit dem Fußteil 6 des Plattenkörpers 1 bilden.

Im Platz- oder Abstandshalter 7 ist eine Gewindebohrung 10 eingebracht, die sich im Verbindungsabschnitt 11 zwischen den seitlichen Schenkeln 9 befindet.

Die Schraube 12 zur Befestigung des Platz- oder Abstandshalters 7 weist einen hin zum Gewindeteil gerichteten, sich konisch verjüngenden Schraubenkopf 13 auf, um eine sichere Befestigung des Platz- oder Abstandshalters 7 am Plattenkörper 1 zu gewährleisten.

Die Platz- oder Abstandshalter 7 können mit Hilfe der Schraube 12 an Standard-Plattenkörpern oder solchen mit unterschiedlich langen Fußteilen 6 befestigt werden, wobei die Höhe H des jeweiligen Platz- oder Abstandshalters 7 der Öffnung des Osteotomie-Keilschnitts entspricht bzw. auf das Korrekturmaß abgestimmt wird.

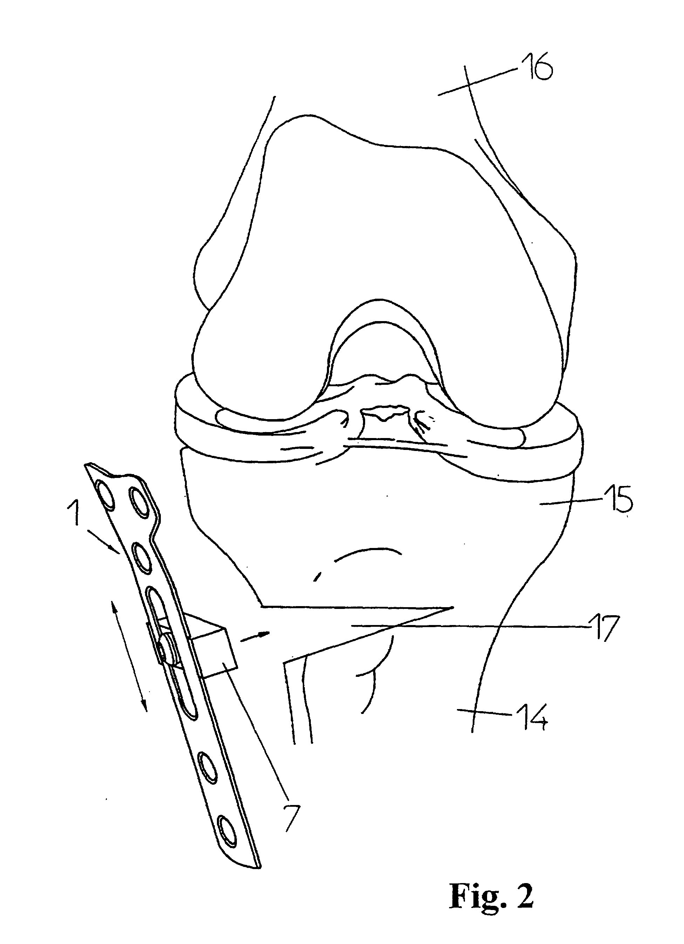

Fig. 2 stellt stark vereinfacht einen Abschnitt der Tibia 14 mit Tibiakopf 15 sowie des Femur 16 mit ausgeführtem Osteotomie-Keil dar.

Der Plattenkörper 1 mit lose vormontiertem Platz- oder Abstandshalter 7 wird mit letzterem voran in die Osteotomie-Keilöffnung 17 eingesetzt, um hiernach eine optimale Position für die Befestigungsbohrungen 5 zu ermitteln.
Grundsätzlich sollen die Befestigungsbohrungen im Kopfteil 4 des Plattenkörpers 1 ihre Lage im Tibiakopf finden, so daß die Folgen von Spontanfrakturen in diesem Bereich gemildert werden.
Nachdem die gewünschte Befestigungsposition ermittelt wurde, werden an sich bekannte selbstschneidende Knochenschrauben zum Fixieren des Plattenkörpers 1 in der Tibia und dem Tibiakopf genutzt. Ebenso erfolgt ein endgültiges Anziehen und Befestigen des Platz- oder Abstandshalters 7 am Plattenkörper 1 mit Hilfe der Schraube 12 und eines geeigneten (nicht gezeigten) Werkzeugs.

### Bezugszeichenliste

- 1: Plattenkörper
- 2: Langloch
- 3: konische Fase
- 4: Kopfteil
- 5: Befestigungsbohrungen
- 6: Fußteil
- 7: Platz- oder Abstandshalter
- 8: Ausnehmung im Platz- oder Abstandshalter
- 9: seitliche Schenkel der U-förmigen Ausnehmung
- 10: Gewindebohrung
- 11: Verbindungsabschnitt
- 12: Schraube für Platzhalter
- 13: Schraubenkopf
- 14: Tibia
- 15: Tibiakopf
- 16: Femur
- 17: Osteotomie-Keil

## Patentansprüche

1. Vorrichtung zur Stabilisierung der Tibia und/oder des Tibiakopfes nach Osteotomie-Eingriffen, bestehend aus einem langgestreckten Plattenkörper mit Bohrungen zur Aufnahme von Knochenschrauben sowie einem quaderförmigen Platz- oder Abstandshalter, wobei der Plattenkörper ein in Längsachsenrichtung verlaufendes Langloch aufweist, der Platz- oder Abstandshalter verschieblich und feststellbar über eine Schraubverbindung im Langloch des Plattenkörpers gehalten ist und der Platzoder Abstandshalter verschiedene, insbesondere Höhenabmessungen aufweist und austauschbar ist,
**dadurch gekennzeichnet, dass**
der Platz- oder Abstandshalter (7) eine U-förmige Ausnehmung (8) aufweist, wobei die seitlichen Schenkel (9) der Ausnehmung (8) den Plattenkörper (1) umgreifen, wodurch eine Verdrehsicherung und Führung gebildet ist.

2. Vorrichtung nach Anspruch 1,
**dadurch gekennzeichnet, dass**
der Platz- oder Abstandshalter (7) eine Gewindebohrung (10) aufweist, die sich im Verbindungsabschnitt (11) zwischen den seitlichen Schenkeln (9) der Ausnehmung (8) befindet.

3. Vorrichtung nach Anspruch 1 oder 2,
**dadurch gekennzeichnet, dass**
das Langloch (2) eine zur Oberseite des Plattenkörpers (1) verlaufende konische Fase (3) aufweist.

4. Vorrichtung nach Anspruch 3,
**dadurch gekennzeichnet, dass**
die Schraube (12) zur Befestigung des Platz- oder Abstandshalters (7) einen sich konisch verjüngenden Schraubenkopf (13) oder Schraubenkopfabschnitt besitzt.

5. Vorrichtung nach einem der vorangegangenen Ansprüche,
**dadurch gekennzeichnet,dass**
der Plattenkörper (1) eine T-Form aufweist, wobei im querliegenden Kopfteil (4) mindestens zwei Befestigungsbohrungen (5) sowie im langgestreckten Fußteil (6) dem Langloch (2) in Längsachsenrichtung benachbart jeweils mindestens eine weitere Befestigungsbohrung (5) ausgebildet ist.

6. Vorrichtung nach Anspruch 5,
**dadurch gekennzeichnet, dass**
die Kopfteilbohrungen (5) der Befestigung des Plattenkörpers (1) im Tibiakopf (15) und dem Stabilisieren der Knochenstruktur des Kopfes selbst dienen.

7. Vorrichtung nach einem der vorangegangenen Ansprüche,
**dadurch gekennzeichnet,dass**
der Querschnitt des Plattenkörpers (1) eine Bogen- oder Kreissegmentform besitzt.

## Claims

1. A device for stabilising the tibia and/or the tibial head after osteotomic surgery, comprising an elongate plate with bores for receiving bone screws, and a cuboidal spacer, wherein the plate has a slot extending in the longitudinal axial direction, the spacer is displaceably held in the slot of the plate and is fixable therein by means of a screw connection, and the spacer has different dimensions, in particular height dimensions, and is exchangeable, **characterised in that** the spacer (7) has a U-shaped recess (8), the lateral arms (9) of the recess (8) engaging round the plate (1) and thereby forming an anti-rotation means and a guide.

2. A device accordirig to claim 1, **characterised in that** the spacer (7) has a threaded bore (10) provided in the connecting portion (11) between the lateral arms (9) of the recess (8).

3. A device according to claim 1 or 2, **characterised in that** the slot (2) has a conical chamfer (3) extending towards the upper surface of the plate (1).

4. A device according to claim 3, **characterised in that** the screw (12) for fixing the spacer (7) has a conically tapering screw head (13) or screw-head portion.

5. A device according to any one of the preceding claims, **characterised in that** the plate (1) is T-shaped, at least two fixing bores (5) being formed in the transverse head part (4), and at least one further fixing bore (5) being formed in the elongate foot part (6) adjacent to the slot (2) in the longitudinal axial direction.

6. A device according to claim 5, **characterised in that** the bores (5) in the head part (4) serve to secure the plate (1) in the tibial head (15) and to stabilise the bone structure of the head itself.

7. A device according to any one of the preceding claims, **characterised in that** the cross-section of the plate (1) has a curved shape or is in the form of a circular segment.

## Revendications

1. Dispositif pour stabiliser le tibia et/ou la tête de tibia suite à des interventions d'ostéotomie, constitué par un corps allongé en forme de plaque présentant des perçages pour recevoir des vis à os ainsi qu'un élément mainteneur/écarteur en forme de parallélépipède, le corps formant plaque présentant un trou oblong s'étendant en direction de l'axe longitudinal, l'élément mainteneur/écarteur étant retenu de façon mobile et susceptible d'être arrêté par une liaison vissée dans le trou oblong du corps formant plaque et l'élément mainteneur/écarteur présentant des dimensions différentes en particulier en hauteur et étant interchangeable,
**caractérisé en ce que** l'élément mainteneur/écarteur (7) présente un évidement en forme de U (8), les branches latérales (9) de l'évidement (8) entourant le corps formant plaque (1), dont résulte un blocage anti-rotation et un guidage.

2. Dispositif selon la revendication 1, **caractérisé en ce que** l'élément mainteneur/écarteur (7) présente un perçage taraudé (10) qui se trouve dans le tronçon de liaison (11) entre les branches latérales (9) de l'évidement (8).

3. Dispositif selon l'une ou l'autre des revendications 1 et 2, **caractérisé en ce que** le trou oblong (2) présente un chanfrein conique (3) qui s'étend vers le côté supérieur du corps formant plaque (1).

4. Dispositif selon la revendication 3, **caractérisé en ce que** la vis (12) destinée à fixer l'élément mainteneur/écarteur (7) possède une tête de vis (13) ou un tronçon de tête de vis qui va en se rétrécissant coniquement.

5. Dispositif selon l'une des revendications précédentes, **caractérisé en ce que** le corps formant plaque (1) présente une forme en T, au moins deux perçages de fixation (5) étant ménagés dans la partie de tête transversale (4) et au moins un autre perçage de fixation respectif (5) étant ménagé dans la partie de pied allongée (6) au voisinage du trou oblong (2) en direction de l'axe longitudinal.

6. Dispositif selon la revendication 5, **caractérisé en ce que** les perçages (5) de la partie de tête servent à la fixation du corps formant plaque (1) dans la tête de tibia (15) et à stabiliser la structure osseuse de la tête elle-même.

7. Dispositif selon l'une des revendications précédentes, **caractérisé en ce que** la section transversale du corps formant plaque (1) possède une forme d'arc ou de segment circulaire.
